# EUROPÄISCHE PATENTANMELDUNG

(11) **EP 1 321 528 A1**
(43) Veröffentlichungstag der Anmeldung: **25.06.2003**
(21) Anmeldenummer: 02028399.0
(22) Anmeldetag: 18.12.2002
(51) Int. Cl.: C12P 41/00

(54) **Vefahren zur kinetischen Racematspaltung von Alkoholen mit einem oder mehreren stereogenen Zentren**

(30) Priorität: 19.12.2001 DE 10164269
(71) Anmelder: ASCA GmbH, Angewandte Synthesechemie Adlershof, 12489 Berlin (DE)
(72) Erfinder: Theil, Fritz, Dr. rer. nat., 12247 Berlin (DE); Sonnenschein, Helmut, Dr. rer. nat., 10245 Berlin (DE)
(74) Vertreter: Uhlemann, Henry, Dipl.-Chem.

(57) **Zusammenfassung**

Die vorliegende Erfindung betrifft ein Verfahren zur kinetischen Racematspaltung von Alkoholen mit einem oder mehreren stereogenen Zentren. Die Erfindung ist insbesondere für die Herstellung pharmazeutischer Wirkstoffe oder Pflanzenschutzmittel anwendbar.
Erfindungsgemäß werden zunächst racemische Alkohole mit fluorierten Acylierungsmitteln in racemische fluorierte Carbonsäureester überführt. Dann wird durch lipasekatalysierte Umsetzung des racemischen fluorierten Carbonsäureesters mit Alkoholen die Fluorphasenmarkierung des schneller reagierenden Enantiomers aufgehoben und die des langsamer reagierenden Enantiomers erhalten. Anschließend werden die Enantiomeren durch Verteilung zwischen organischer und fluoriger Phase extraktiv getrennt.

## Beschreibung

Die vorliegende Erfindung betrifft ein Verfahren zur kinetischen Racematspaltung von Alkoholen mit einem oder mehreren stereogenen Zentren. Die Erfindung ist insbesondere für die Herstellung pharmazeutischer Wirkstoffe oder Pflanzenschutzmittel anwendbar.

Die lipasekatalysierte kinetische Racematspaltung von Alkoholen durch enantiomerselektive Veresterung bzw. durch enantiomerselektive Hydrolyse oder Alkoholyse chiraler Carbonsäureester ist eine wohl etablierte Methode in der organischen Synthese (F. Theil Chem. Rev. 1995, 95, 2203-2227, U. T. Bornscheuer, R. J. Kazlauskas, Hydrolases in Organic Synthesis, Wiley-VCH, Weinheim, 1999). Das langsamer reagierende Enantiomer bleibt dabei als Alkohol bzw. Carbonsäureester zurück, während das schneller reagierende Enantiomer als Carbonsäureester oder Alkohol anfällt. Üblicherweise werden Alkohol und Carbonsäureester chromatographisch getrennt (F. Theil et al. J. Org. Chem. 1994, 59, 388-393).
Einfachere Trennungen werden nur erreicht, wenn sich beispielsweise Alkohol und Ester in ihren Löslichkeitseigenschaften soweit unterscheiden, daß eine extraktive Trennung zwischen wäßriger und organischer möglich ist, eine Verfahrensweise, die jedoch nur in Ausnahmefällen realisiert werden kann (P. Stead et al. Tetrahedron: Asymmetry 1996, 7, 2247-2250).
Eine weitere Möglichkeit zur Trennung von Ester und Alkohol ist dann gegeben, wenn der entstandene Ester sauer ist, was beispielweise durch die Veresterung mit cyclischen Carbonsäureanhydriden realisierbar ist. Allerdings ist auch das keine allgemein anwendbare Methode, weil cyclische Anhydride keine optimalen Acyldonoren für lipasekatalysierte Veresterungen sind. Darüber hinaus verringern saure Verbindungen die Lipaseaktivität (B. Berger et al. Tetrahedron: Asymmetry 1990, 1, 541-546, U. T. Bornscheuer, R. J. Kazlauskas, Hydrolases in Organic Synthesis, Wiley-VCH, S. 44-47, Weinheim, 1999).

Weiterhin wurde ein Verfahren zur lipasekatalysierten Racematspaltung vorgeschlagen, bei dem racemische Alkohole mit fluorierten Acylierungsmitteln oder fluorierter Carbonsäureester racemischer Alkohole mit Wasser umgesetzt werden, wobei eine Fluorphasenmarkierung des schneller oder langsamer reagierenden Enantiomers erreicht wird und die Enantiomeren anschließend durch Verteilung zwischen organischer und fluoriger Phase extraktiv getrennt werden (F. Theil, H. Sonnenschein PCT/DE00/004536). Hydrolysen können jedoch bei bestimmten Substraten gegenüber Umsetzungen im organischen Medium Nachteile hinsichtlich Stabilität von Substrat und/oder Produkt im wässrigen Medium, Reaktionsführung, Isolierung der Produkte und Wiedergewinnung der Lipasen aufweisen. Verfahren zur Acylierung racemischer Alkohole umgehen zwar diese Schwierigkeiten. Es ist aber bekannt, dass die Acylierung von Alkoholen, die Hydrolyse und die Alkoholyse von Carbonsäureestern mit jeweils unterschiedlicher Selektivität verlaufen können (M. Ranchoux et al. Tetrahedron: Asymmetry: 1998, 9, 581-587, K. Takabe et al. Tetrahedron Lett. 2000, 41, 9859-9863) und somit Alkoholysen bei bestimmten Substraten nicht nur vorteilhaft im Vergleich zu Hydrolysen sondern auch gegenüber Acylierungen sind.

Es ist deshalb Aufgabe der Erfindung, ein verbessertes Verfahren zur Spaltung von Alkoholen mit einem oder mehreren stereogenen Zentren zu schaffen.

Erfindungsgemäß wird die Aufgabe durch ein Verfahren zur kinetischen Racematspaltung von Alkoholen mit einem oder mehreren stereogenen Zentren gelöst, bei dem zunächst racemische Alkohole mit fluorierten Acylierungsmitteln in racemische fluorierte Carbonsäureester überführt werden. Dann wird durch lipasekatalysierte Umsetzung des racemischen fluorierten Carbonsäureesters mit Alkoholen die Fluorphasenmarkierung des schneller reagierenden Enantiomers aufgehoben und die des langsamer reagierenden Enantiomers erhalten. Anschließend werden die Enantiomeren durch Verteilung zwischen organischer und fluoriger Phase extraktiv getrennt.

Erfindungsgemäß werden racemische Alkohole mit einem oder mehreren stereogenen Zentren mit perfluorierten Acylierungsmitteln in einen Ester der Formel I,

R-(CH₂)ₙ-COOCHR¹R² (I)

in der
- R: ein perfluorierter Alkylrest wie - (CF₂)ₘ-CF₃, wobei m eine ganze Zahl von 3 bis 18 sein kann,
oder
ein perfluorierter aromatischer Rest wie C₆F₄X ist
und
- X: Fluor oder ein perfluorierter Alkylrest ist,
- R¹, R²: alkyl, alkenyl, aryl oder heteroaryl sind
und
- n: eine ganze Zahl von 0 bis 4 sein kann,
überführt und anschließend in bekannter Weise mit esterspaltenden Enzymen, vorzugsweise Lipasen, einer enantiomerselektiven Alkoholyse mit einem Alkohol der Formel II,

R³-OH (II)

in der
- R³: ein aliphatischer Alkylrest aus ein bis zwölf Kohlenstoffatomen, ein cycloaliphatischer Alkylrest aus vier bis acht Kohlenstoffatomen oder ein Benzyl-oder arylsubstituierter Benzylrest ist,
unterworfen.

Erfindungsgemäß wird dabei gemäß Schema 1 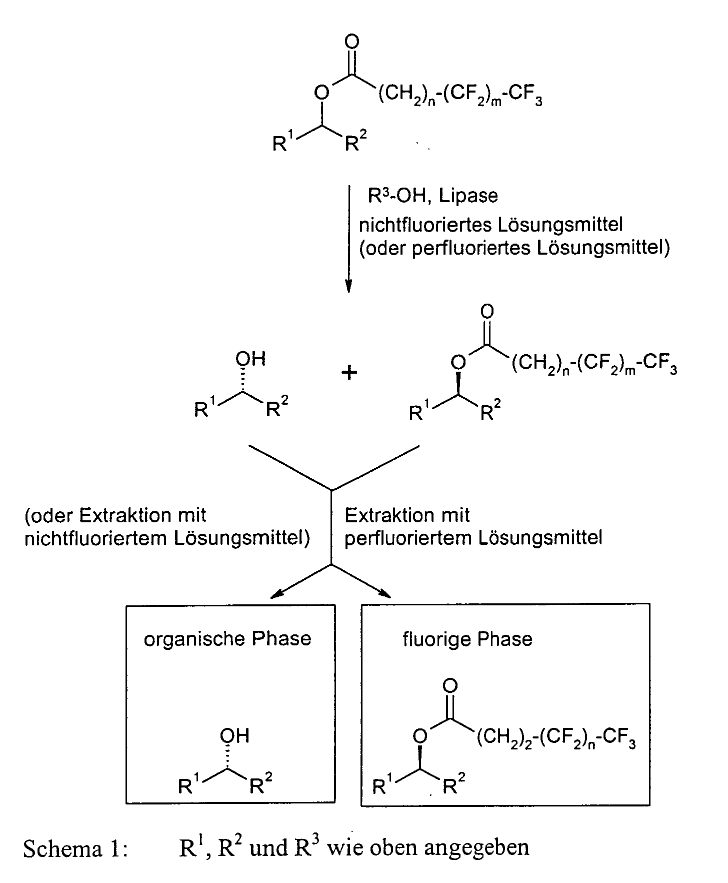 in Gegenwart eines esterspaltenden Enzyms, vorzugsweise einer Lipase, der perfluorierte Rest von dem schneller reagierenden Enantiomer abgepalten, wodurch dieses Enantiomer in fluorigen Lösungsmitteln nicht mehr löslich ist. Bei der anschließenden Verteilung der Reaktionsprodukte zwischen organischer und fluoriger Phase befinden sich erfindungsgemäß der freie Alkohol in der organischen und der nichtgespaltene Ester in der fluorigen Phase.

Erfindungsgemäß wird die Alkoholyse mit einer Lipase mikrobieller, pflanzlicher oder tierischer Herkunft entweder in einem für diese Reaktionen üblichen Lösungsmitteln, wie aliphatischen und aromatischen Kohlenwasserstoffen, Ethern, tertiären Alkoholen oder auch Chlorkohlenwasserstoffen durchgeführt. Anschließend wird das perfluorierte Enantiomer mit einem perfluoriertem Lösungsmittel, welches mit dem nichtfluoriertem organischen Lösungsmittel nicht mischbar ist, extrahiert. Alternativ werden die lipasekatalysierten Reaktionen in einem perfluorierten Lösungsmittel durchgeführt und anschließend das nichtfluorierte Enantiomer mittels nichtfluoriertem organischen Lösungsmittel extrahiert.
Erfindungsgemäß können die lipasekatalysierten kinetischen Racematspaltungen auch in einem bei Raum- oder niedrigerer Temperatur nicht mischbaren Zweiphasensystem aus organischem und fluorigem Lösungsmittel durchgeführt werden. Die Phasenhomogenisierung während der chemischen Reaktion wird durch Erwärmen realisiert. Die Phasen- und damit verbundene Produkttrennung wird durch Abkühlung des Reaktionsgemisches unter die Phasenmischungstemperatur erreicht.
Auf diese Weise ist die Trennung der Enantiomeren in guten Ausbeuten durchführbar.

Anhand nachfolgender Ausführungsbeispiele wird die Erfindung näher erläutert:

### Ausführungsbeispiel 1

Ein Lösung von racemischem 4,4,5,5,6,6,7,7,8,8,9,9,10,10,11,11,11-Heptadecafluorundecansäure-1-phenylethylester (1,79 g, 3 mmol) und n-Butanol (0.89 g, 12 mmol) in Acetonitril (70 mL) wird mit Candida antarctica B Lipase (5 g) versetzt und gerührt, bis 50 % des Esters umgesetzt sind. Das Enzym wird abfiltriert und das Filtrat i. Vak. zur Trockene eingeengt. Der Rückstand wird in Methanol (10 ml) gelöst und die Lösung sechsmal mit n-Perfluorhexan extrahiert. Die organische Phase enthält (R)-1-Phenylethanol mit einem Enantiomerenüberschuß von > 95 %. Die fluorige Phase enthält (S)-4,4,5,5,6,6,7,7,8,8,9,9,10,10,11,11,11-Heptadecafluorundecansäure-1-phenylethylester mit einem Enantiomerenüberschuß von > 95 %.

### Ausführungsbeispiel 2

Ein Lösung von racemischem 4,4,5,5,6,6,7,7,8,8,9,9,10,10,11,11,11-Heptadecafluoroundecansäure-indan-1-yl ester (1,82 g, 3 mmol) und n-Butanol (0.89 g, 12 mmol) in Acetonitril (70 mL) wird mit Candida antarctica B Lipase (5 g) versetzt und gerührt bis 50 % des Esters umgesetzt sind. Das Enzym wird abfiltriert und das Filtrat i. Vak. zur Trockene eingeengt. Der Rückstand wird in Methanol (10 ml) gelöst und die Lösung sechsmal mit n-Perfluorhexan extrahiert. Die organische Phase enthält (R)-1-Indanol mit einem Enantiomerenüberschuß von > 95 %. Die fluorige Phase enthält (S)-4,4,5,5,6,6,7,7,8,8,9,9,10,10,11,11,11-Heptadecafluoro-undecansäure-indan-1-yl ester mit einem Enantiomerenüberschuß von ≥ 90 %.

### Ausführungsbeispiel 3

Ein Lösung von racemischem 4,4,5,5,6,6,7,7,8,8,9,9,10,10,11,11,11-Heptadecafluor-undecansäure 1-methyl-3-trimethylsilanyl-prop-2-inyl ester (1.85 g, 3 mmol) in Acetonitril (70 mL) und n-Butanol (0.89 g, 12 mmol) wird mit Candida antarctica B Lipase (5 g) versetzt und gerührt bis ca. 40 % des Esters umgesetzt sind. Das Enzym wird abfiltriert und das Filtrat i. Vak. zur Trockene eingeengt. Der Rückstand wird in Methanol (10 ml) gelöst und die Lösung sechsmal mit n-Perfluorhexan extrahiert. Die organische Phase enthält (R)- 4-Trimethylsilanyl-but-3-in-2-ol mit einem Enantiomerenüberschuß von > 99 %. Die fluorige Phase enthält (S)- 4,4,5,5,6,6,7,7,8,8,9,9,10,10,11,11,11-Heptadecafluor-undecansäure 1-methyl-3-trimethylsilanyl-prop-2-inyl ester mit einem Enantiomerenüberschuß von 68 %.

## Patentansprüche

1. Verfahren zur kinetischen Racematspaltung von Alkoholen mit einem oder mehreren stereogenen Zentren, bei dem zunächst racemische Alkohole mit fluorierten Acylierungsmitteln in racemische fluorierte Carbonsäureester überführt werden
**dadurch gekennzeichnet, daß**
durch lipasekatalysierte Umsetzung des racemischen fluorierten Carbonsäureesters mit Alkoholen die Fluorphasenmarkierung des schneller reagierenden Enantiomers aufgehoben und die des langsamer reagierenden Enantiomers erhalten bleibt und daran anschließend die Enantiomeren durch Verteilung zwischen organischer und fluoriger Phase extraktiv getrennt werden.

2. Verfahren nach Anspruch 1, **dadurch gekennzeichnet, daß** zur kinetischen Racematspaltung von Alkoholen mit einem oder mehreren stereogenen Zentren racemische Alkohole mit fluorierten Acylierungsmitteln in ihre Ester der Formel **I**,
R-(CH₂)ₙ-COOCHR¹R² (**I**)
wobei
R ein perfluorierter Alkylrest wie - (CF₂)ₘ-CF₃, wobei m eine ganze Zahl von 3 bis 18 sein kann,
oder
ein perfluorierter aromatischer Rest wie C₆F₄X
und
X Fluor oder ein perfluorierter Alkylrest sind,
R¹, R² alkyl, alkenyl aryl oder heteroaryl sind
und
n eine ganze Zahl von 0 bis 4 sein kann,
überführt werden, diese Ester anschließend einer enantiomerselektiven Alkoholyse mit einem Alkohol der Formel **II**,
R³-OH (**II**)
in der
R³ ein aliphatischer Alkylrest aus ein bis zwölf Kohlenatomen, ein cycloaliphatischer Alkylrest aus vier bis acht Kohlenstoffatomen oder ein Benzyl-oder arylsubstituierter Benzylrest ist,
unterworfen werden
und die erhaltenen Enantiomeren durch extraktive Verteilung zwischen organischer und fluoriger Phase voneinander getrennt werden.

3. Verfahren nach Anspruch 1, **dadurch gekennzeichnet, daß** die Lipasen mikrobieller, pflanzlicher oder tierischer Herkunft sind.

4. Verfahren nach Anspruch 2 oder 3, **dadurch gekennzeichnet, daß** die Alkoholyse in aliphatischen oder aromatischen Kohlenwasserstoffen, Ethern, tertiären Alkoholen oder Chlorkohlenwasserstoffen durchgeführt wird und die Extraktion des fluorierten Enantiomeren mit einem perfluorierten Lösungsmittel erfolgt.

5. Verfahren nach Anspruch 2 oder 3, **dadurch gekennzeichnet, daß** die Alkoholyse in einem perfluorierten Lösungsmittel und die Extraktion mit einem nichtfluorierten Lösungsmittel durchgeführt werden.

6. Verfahren nach Anspruch 2 oder 3, **dadurch gekennzeichnet, daß** die lipasekatalysierte Alkoholyse in einem Zweiphasensystem aus organischer und fluoriger Phase durchgeführt wird, wobei die Phasenhomogenisierung während der Reaktion durch Erwärmen erreicht wird, und nachfolgend die Phasen- und damit Produkttrennung durch Abkühlen des Reaktionsgemisches unterhalb der Phasenmischungstemperatur vorgenommen wird.

7. Verfahren nach Anspruch 4 bis 6, **dadurch gekennzeichnet, daß** die bei der lipasekatalysierten Alkoholyse verwendeten Lösungsmittel nach Abbruch der Reaktion abdestilliert und anschließend eine Verteilung der Produkte zwischen organischer und fluoriger Phase erfolgt.
